Europäisches Patentamt

(19) European Patent Office

Office européen des brevets

(11) Publication number: **0 256 821**
**A2**

## (12) EUROPEAN PATENT APPLICATION

(21) Application number: 87307049.4

(22) Date of filing: 07.08.87

(51) Int. Cl.⁴: **A 61 K 7/48**

(30) Priority: 11.08.86 CA 515681

(43) Date of publication of application:
24.02.88 Bulletin 88/08

(84) Designated Contracting States:
AT BE CH DE ES FR GB GR IT LI LU NL SE

(71) Applicant: **ADVANCED ORAL HEALTHCORP LIMITED**
**3220 Park Place 666 Burrard Street**
**Vancouver British Columbia V6C 2X8 (CA)**

(72) Inventor: **Roydhouse, Richard R.**
**4917 College Highroad**
**Vancouver British Columbia V6T 1G7 (CA)**

(74) Representative: **Crampton, Keith John Allen et al**
**D YOUNG & CO 10 Staple Inn**
**London WC1V 7RD (GB)**

(54) Body lubricating and cleansing fluid.

(57) A composition comprises lecithin, an edible oil and a fatty acid in which the ratio of fatty acid to lecithin is such that the composition has the ability to render a hydrophobic surface hydrophilic. The composition is useful as a body lubricant and cleanser and differs from the prior art in its ability to render a hydrophobic surface hydrophilic.

EP 0 256 821 A2

**Description**

## BODY LUBRICATING AND CLEANSING FLUID

FIELD OF THE INVENTION

This invention relates to novel lecithin containing solutions or stable dispersions which act as lubricants or cleansers.

DESCRIPTION OF THE PRIOR ART

A human or animal body lubricant or cleanser should not be toxic or interfere with the metabolic processes of the body surfaces. In contrast to machines, the surfaces of human and animal bodies are replaced steadily (eg. skin) and the surfaces are continually coated or washed with special fluids such as sweat and sebaceous secretions on skin, various secretions on mucous membranes of body cavities such as saliva in the mouth, pharynx and oesophagus. These natural fluids act as cleansing agents and lubricants and can indirectly control microbial populations on surfaces.

The surface maintenance of human bodies and cavities requires a certain equilibrium with water. For instance saliva, a mouth lubricant, is not totally soluble in water. Such is also the case with, for example, chest sputum and nasal secretions. Mucous membrane surfaces when air-dried are hydrophobic i.e. have no affinity for water. However, these surfaces are covered continuously by secretions such as saliva which are water immiscible but hydrophilic i.e. have an affinity for water. Secretions alter the water repellency of living surfaces; dissolution by water or hydration of surfaces can be prevented without eliminating the ability to absorb water or waterborne materials. It is the absence of secretions that make body surfaces hydrophobic and thus dry or non lubricated surfaces.

In human lubrication, smooth surfaces are not the norm, consider for example, the tongue, lips and gums. Because of the corrugated and irregular surfaces, the ability of a lubricant-cleanser to flow, cover and remain attached to a tissue substrate is critical.

Film forming lubricants previously used on bodily surfaces, for example petrolatum and lanolin, have not been developed to mimic natural body secretions. Petrolatum, for example, applied to tissues increases debris pickup, interferes with water transpiration and does not provide a covering of a nature that lasts. Nor do the waxes and oils used to make materials for lip protection. Grad et al. in the Journal of the Canadian Dental Association Volume 4, pages 296-300, 1985 shows that prior art materials for alleviating dry mouth disorders, i.e. artificial salivas, are inadequate for this purpose.

Generally formulations for lubrication and cleansing of human and animal tissue or non-animal hydrophobic surfaces require the following features:

1. A capacity to form a tenacious film.
2. A capacity to resist water.
3. A capacity to remain on the surface despite passing diluents.
4. Minimum interference with normal water and gas transportation due to fouling or stripping of bodily secretions.
5. No adverse effect on normal metabolic processes in the underlying layer or organ to which they are applied.
6. Tuneable viscosity, either high or low, to suit the particular application.
7. Form a solution or stable dispersion to prevent phase separation of ingredients in storage or during application.
8. Have inoffensive smell or odour, and not cause pain (eg. stinging) when applied.
9. Reasonable cost of production.

The prior art has developed a variety of lecithin containing formulations for cosmetic purposes but these formulations lack one or more of the above desirable attributes. For example Genieyz in UK patent 2,004,741 describes lecithin containing formulations containing chloresterol, an unsaturated fatty acid, such as linoleic acid, and a phospho aminolipid (eg. lecithin) in a solvent such as ethanol. The ethanol is used to solubilize the lecithin containing mixture and is the major component of the formulation eg. 94% by weight in Example 1. The Hazardous Chemicals data book indicates that ethyl alcohol vapour causes irritation of eyes, nose and throat. Headache and drowsiness may occur and the liquid can cause intoxication at such high ethanol levels. Clearly lecithin containing formulations with such a high alcohol content can cause drying and other adverse effects on normal metabolic processes especially if administered orally. In addition, a preferred composition having a linoleic acid component would have a relatively high manufacturing cost.

Tutsky (Published International Patent Application No. 85/00746) describes a skin conditioning composition for use in shaving which contains a phospholipid such as lecithin, vitamin E, wheat germ oil, seed and vegetable oils. Tutsky's examples indicate low lecithin content (i.e. 5-12% by weight) and very high oil contents e.g. 88-95% by weight. The purpose of the high oil content is clearly to dissolve the lecithin.

Lecithin consists of glycerol combined with two fatty acid radicals, phosphoric acid and choline. According to the Merck Index (9th edition) soybean lecithin contains palmitic acid, stearic acid, palmitoleic acid, oleic acid, linolenic acid, and linoleic acid. Cottonseed, egg and groundnut lecithins also contain these fatty acids. These fatty acids are chemically bonded to form esters within the lecithin. This is evidenced by the fact that treatment of lecithin with aqueous alkali is required to split off the fatty acid species from the lecithin. The "free" fatty acid content of the lecithin is low at approximately 1% by weight. Lecithin is insoluble in water. However it is soluble in mineral oils or alcohols. It is practically insoluble in cold vegetable and animal oils.

However the term "lecithin" has both a broad and narrow meaning. The narrow meaning applies to a phosphatidylcholine. The broad meaning applies to any of the raw or crude mixtures such as those derived from oil seeds (for example soybeans and rape seeds) or yolk of egg, which contain phosphatides. Pure or plastic lecithin is so expensive that it is preferred to use lecithin in its raw forms. Thus the lecithin suitable for use in the present invention includes any of the commercially available lecithin compositions such as Epikuron 135 (Trade Mark) sold by the Lucas Meyer Company. A readily available and inexpensive source of lecithin is soybeans.

The prior art does not provide a lecithin containing solution or readily flowable stable dispersion which can act as a lubricant or cleanser especially on living tissue, which does not require the use of excessive amounts of irritating solvents (e.g. $\geq 94\%$ ethanol) or can coat hydrophobic surfaces in a manner which mimics normal body secretions.

Accordingly the present invention concerns a composition able to enhance the natural cleansing, lubricating and bacteriostatic properties of human and animal secretions as well as lubrication and cleaning of hydrophobic surfaces (eg. marine structures under water).

In this regard it has been found that a formulation deemed desirable by Genieyz in U.K. patent 2,004,741 and containing 3% oleic acid, 1% cholesterol, F, from Lucas Meyer and Sohn GMBH) and 94% ethanol would not flow underwater and thereby coat a submersed hydrophobic surface such as parafilm.

Further Tutsky in published International patent 85/00746 is, as remarked above using oil to dissolve the lecithin rather than enhance its ability to wet and coat hydrophobic surfaces, especially under water.

## SUMMARY OF THE INVENTION

The invention provides novel lubricant-cleaners that achieve cleansing via displacement of the adhering debris by the film of the lubricant. This is not the mode of cleansing achieved by detergents and soaps which remove bodily fluids (e.g. lipids) and decrease lubrication.

According to the present invention plastic lecithin (oil free) can be dissolved at room temperature in a mixture of edible oils plus free fatty acid and commercial lecithin (oil containing) can be dissolved at room temperature with or without prior heating in free fatty acid to form either homogeneous solutions or stable dispersions. Such solutions and their variants can be used in a wide variety of applications involving lubrication and cleansing of living and non-living hydrophobic surfaces. These unique formulations have the ability to form tenacious films upon hydrophobic surfaces despite their affinity for water. The formulations do not require the use of potentially toxic petroleum derived oils (e.g. mineral oils) or excessive amounts of solvents such as ethanol.

Although it has been documented that plastic lecithins are converted to fluid forms by adding 2-5% fatty acids or carriers such as soybean oil (Encyclopedia of Food Science Volume 3, 1978, page 463), the prior art has failed to develop cleansers or lubricants which have appropriate amounts of lecithin in combination with edible oils plus free fatty acids. For instance there has been no indication that it would be desirable to combine a saturated fatty acid such as myristic acid (solid at room temperature) together with commercial lecithin to form a liquid solution at room temperature. We suggest that the Encyclopedia of Food Science reference cited above refers to unsaturated fatty acids (liquids at room temperature) only. Also this reference discusses plastic lecithin (oil free) and not commercial lecithin (oil containing). The present invention shows that use of very high fatty acid contents (e.g. 10% myristic acid in 90% commercial lecithin) results in formulations with the outstanding, lubricating-cleansing characteristics previously deemed desirable. It is suggested that the excess free fatty acid alters the hydrophilic-hydrophobic properties of lecithin in a previously undiscovered way.

Accordingly the present invention is a composition comprising lecithin; an edible oil; a fatty acid, the ratio of fatty acid to lecithin in the composition being such that the composition has the ability to render a hydrophobic surface hydrophilic.

In a preferred embodiment the ratio of free fatty acid to lecithin is within the range of about 4.5:1 to 11:1.

In this application the term "free fatty acid" in a composition of the invention means a fatty acid added to the composition plus the fatty acid present in the lecithin and non-covalently bonded, that is the approximately 1% non-ester fatty acid referred to above.

In calculating ratios the lecithin is given as pure or plastic lecithin, as discussed above, even though the lecithin used to make the composition is usually a commercially available lecithin containing, typically, about 50% pure or plastic lecithin.

## DESCRIPTION OF THE PREFERRED EMBODIMENTS

The following Tables illustrate the wide range of attractive lubricant or cleanser formulations using the high free fatty acid to lecithin ratio characteristics.

TABLE 1:  Lecithin – Edible Oil – Free Fatty Acid Formulations

## TABLE 2

Lecithin - Edible Oil - Free Fatty Acid Formulations

| Formula Code | Ingredients[1] | Suggested Applications | Solution (S) or Stable Dispersion (SD) |
|---|---|---|---|
| A[2] | 50% plastic lecithin, 49% edible oil, 1% free fatty acid. | Not recommended. | S |
| B[2] | Commercial lecithin (eg soybean containing 50% plastic lecithin, 49% soy oil, 1% free fatty acid). | Not recommended. | S |
| C[2] | >90% commercial lecthin, <10% free fatty acid (eg 45% plastic lecithin, 45% soy oil, 10% free fatty acid). | Additive for gums, lozenges, waxes, lubricant cleanser for hydrophobic surfaces (eg cooking utensils and marine structures, living tissue). | S |
| D | 6-12% propylene glycol, Tween 80 @ 2.5 times % Formula C, % balance as Formula C. | Rubbing lubricant, cutting wet wood, foam. | S |
| E | >29% Formula "C", <71% ethanol. | Dental plaque remover, breath freshener, aerosol. | S |
| F | <29% Formula "C", >71% ethanol. | Not recommended. | Unstable |
| G[6] | % Tween 80 @ 2.5 times Formula C, % balance as Formula C. | High viscosity lubricant, lubricant for cutting wet objects. | S |
| H[5] | Formula D plus water. | Mouthwash, plaque remover, breath freshener. | S |
| I | Formula E plus water. | Lubricant for dry living tissues. | SD |
| J | Formula F plus water. | Not recommended. | Unstable |
| K | Formula G blended with ethanol by heating followed by cooling to room temperature. | Aerosol for oral lubrication and cleaning. | S |
| L[5] | Formula K plus water. | Low viscosity lubricant, mouthwash, plaque remover, breath freshener. | S |
| M[4] | Formula K plus glycerol. | High viscosity lubricant, for surgical purposes. | Two phase solution |
| N | Formula E plus glycerol. | Burn treatment, lubrication of body cavities. | SD |
| O | Formula E plus cod liver oil instead of oil used in C. | Vitamin enriched burn & wound treatment, low viscosity high penetration lubricant-cleanser for body cavities. | SD |
| P[5] | Formula K plus water plus glycerol. | Lubricant cleanser, for douches. | S |
| Q | Formula N plus soap. | Shaving, and body cleansing. | SD |
| R | Formula N plus anaesthetic. | Therapeutic, hospital use, carrier for therapeutics. | SD |
| S | Formula N plus bacteriostat. | Cleanser - lubricant for health care professionals, for handwashing. | SD |
| T | Formula D plus glycerol. | Oral Cleanser. | S |
| U[5] | Formula D plus glycerol plus water. | Oral Cleanser. | S |

Notes

1. Formulas described do not exclude minor additions of other aqueous or non-aqueous flavours, sweeteners, bacteriocides, pharmaceuticals, vitamins, preservatives (eg. antioxidants) and other therapeutic materials as desired for special effects.
2. Edible oil refers to fish oils (as in formula O), vegetable oils or derived from plant or animal origin (eg. whale oil)
3. Ethanol refers to 95% ethanol and 5% water in all examples.
4. The addition of 1% water (See Formula P) creates a stable dispersion, while 5% added water creates a solution.
5. Water addition allows inclusion of inorganic salts such as fluorides and metaphosphates.
6. Tween 80 is a trade mark for a surfactant derived from polyoxyethylene.

The following examples illustrate typical lecithin formulations having desirable properties for a variety of applications. All percentages given are by weight.

Example 1: A "C" Formula

This formula contained 90% commercial soybean lecithin (Lucas Meyer Epikuron 135 F) blended with 10% oleic acid at 54°C and then cooled to room temperature. It can also be made without the addition of heat but heat greatly speeds up solution of lecithin with a saturated fatty acid. The "C" formula was found to wet parafilm (i.e. did not form beads on the surface) under water whereas the commercial lecithin on its own (i.e. Formula "B") did not wet the parafilm under water (i.e. formed beads on the surface). Parafilm is a trade mark for a paraffin wax coated surface useful to test surface tension characteristics of a fluid.

Example 2: An "N" Formula

This formula contained 82.53% glycerol, 6.72% commercial soybean lecithin, 0.67% myristic acid, 10.08% ethanol (95% grade). This mixture is a stable dispersion.

Example 3: An "H" Formula

This formula contained 9.00% propylene glycol, 1.71% com mercial soybean lecithin, 0.17% myristic acid, 0.22% flavour, 0.29% sweetener, 4.63% Tween 80, .10% glycerol mono oleate, 4.77% urea, 0.75% sodium hexametaphosphate and 78.46% water.

Example 4: A "P" Formula

This formula contained 5.00% glycerol 1.71% commercial soybean lecithin, 0.17% myristic acid, 12.15% ethanol, 0.22% flavour, 0.29% sweetener, 4.63% Tween 80, 4.77% urea, 0.75% sodium hexametaphosphate and 70.31% water.

Example 5: A "Q" Formula

This formula contains 60.31% glycerol, 5.20% commercial soybean lecithin, 0.52% myristic acid, 7.79% ethanol, 3.81% cetyl alcohol, 1.42% fragrance, 2.32% sodium lauryl sulphate, 2.71% urea, 14.11% water, 1.59% stearic acid, 0.22% sodium hydroxide).

Example 6: An "R" Formula

This formula illustrating a lubricant cleanser with a carrier of anesthetic for therapeutic purposes was made from 79.03% glycerol, 10.08% ethanol, 6.72% lecithin, 0.67% myristic acid, 3.50% urea (call this Z). Formula Z (95%) was mixed with 5% benzocaine to give the "R" formulation.

Example 7: An "S" Formula

This formula illustrating a lubricant-cleanser containing a bacteriostat suitable for handwashing (e.g. hospitals) contained 55.45% glycerine, 23.23% ethanol, 7.89% Hibitane (a 20% chlorhexidine gluconte solution), 6.50% soybean lecithin (Lucas Meyer Epikuron 135 F), 3.25% Sterling DEA (diethanol cocoamide), 3.03% urea and 0.65% myristic acid. Another "S" formulation not containing chlorhexidene gluconate contained 60.55% glycerin, 25.00% ethanol, 7.00% Epikuron, 135F soybean lecithin, 3.50% Sterling DEA, 3.25% urea an 0.7% myristic acid.

Example 8: An "O" Formula

This formula includes a fish oil and contained 2.92% plastic lecithin, 2.92% cod liver oil, 0.58% myristic acid, 8.77% ethanol, 64.81% glycerol and 20.00% water.

Example 9: A "D" Formula

This formula contained 10.95% soy lecithin (Epikuron 135 F), 1.09% myristic acid, 57.66% propylene glycol, 0.64% glyceryl mono-oleate and 29.66% Tween 80.

Example 10: An "N" Formula

This formula contained 6.72% soy lecithin (Epikuron 135 F), 0.67% myristic acid, 10.08% ethanol and 82.53% glycerol.

Example 11: An "M" Formula

This formula was made in 3 stages. Initially 1.71 parts of soy lecithin (Epikuron 135 F), 0.17 parts of myristic acid and 4.91 parts of Tween 80 were blended at 54°C for 7 minutes followed by cooling and addition of 15 parts ethanol, 0.22 parts flavouring (e.g. cherry oil) and 0.30 parts rhubarb extract then a mix containing: 72.64 parts glycerol, 4.76 parts urea, 0.27 parts aspartame, 0.02 parts sodium cyclamate. In the third stage 99 or 95 parts of the above blend was mixed with 1 or 5 parts water respectively to form a stable dispersion or solution respectively.

Example 12. A "L" Formula

This formula was made in 2 stages. Initially 1.71 parts of soy lecithin (Epikuron 135 F), 0.17 parts myristic acid, and 4.91 parts of Tween 80 were blended at 54°C for 7 minutes followed by cooling and addition of 15.00 parts ethanol, 0.22 parts flavouring, 0.3 parts rhubarb extract, then added to a mix containing: 71.84 parts water, 0.75 parts sodium hexametaphosphate, 0.05 parts sodium fluoride, 4.76 parts urea, 0.27 parts aspartame and 0.02 parts sodium cyclamate.

### Example 13: An "E" Formula
This formula, good for producing an aerosol, contained 26.38% soy lecithin (Epikuron 135 F), 2.62% myristic acid and 71% ethanol.

### Example 14: An "I" Formula
This formula contained 6.72% soy lecithin (Epikuron 135 F), 0.67% myristic acid, 10.03% ethanol and 82.53% water.

### Example 15: A "G" Formula
This formula, good for producing a foam, contained 25.18% soy lecithin, (Epikuron 135 F), 2.5% myristic acid and 72.31% Tween 80.

### Example 16: A "K" Formula
This formula, good for producing an aerosol, contained 7.85% soy lecithin (Epikuron 135 F), 0.78% myristic acid, 22.53% Tween 80 and 68.84% ethanol. All components except ethanol were heated, blended and cooled before addition of the ethanol.

Further tests were carried out to show how much the viscosity and wetting behaviour of plastic lecithin and commercial lecithin could be altered depending on the free fatty acid content of the mixture.

### Example 17: Wetting Experiment
Commercial lecithin - myristic acid blends containing 4.6 - 6.8% myristic acid were found to be barely able to wet parafilm, a hydrophobic surface, under water (Table 3). However a commercial lecithin - myristic acid blend containing 9.1% myristic acid did wet very well parafilm under water. Note that the commercial lecithin contained soybeam oil (see Formula "C"). Degree of wetting as measured by contact angles of various formulations under water is shown in Table 3 below.

## TABLE 3

Effect of Free Fatty Acid on Lecithin Viscosity and Wetting
of Hydrophobic Surface (Parafilm) Under Water

| Formula | Mean Contact Angle | Number of Tests | Standard Deviation | Contact or "Wetting" | Viscosity in Centipoise |
|---|---|---|---|---|---|
| 100% Commercial Lecithin (Plastic lecithin 50% Soy oil 49% Free fatty acid 1%) | 174 | 5 | 3.7 | No | 1308 |
| 98.5% Commercial Lecithin 1.5% myristic acid | 168 | 5 | 1.8 | No | 1221 |
| 96.6% Commercial Lecithin 3.4% myristic | 123 | 5 | 16 | Borderline | 1027 |
| 95.4% Commercial Lecithin 4.6% myristic acid | 111 | 5 | 7.8 | Borderline | 982 |
| 93.2% Commercial Lecithin 6.8% myristic acid | 107 | 5 | 3.4 | Yes | 909 |
| 90.9% Commercial Lecithin 9.1% myristic acid | 89 | 5 | .1.4 | Yes | 780 |

To demonstrate the use of other fatty acids the following table is included. The compositions tested are all N compositions - see Example 10 above. For comparison the Example 10 composition is included:

## TABLE 4

Contact Angles on Paraffin Film with different formulations
using various fatty acids.

| Fatty Acid Instead of Myristic Acid | Contact Angle upon Paraffin Film | | | Contact Angle of water on coated film | | |
|---|---|---|---|---|---|---|
| | Mean | s.d. | n | Mean | s.d. | n |
| Lauric acid | 38 | 4 | 6 | 4 | 1 | 5 |
| Palmitic acid | 36 | 3 | 5 | 6 | 2 | 4 |
| Stearic acid | 39 | 4 | 6 | 5 | 3 | 4 |
| Myristic acid | 40 | 2 | 6 | 5 | 3 | 9 |

This simple trial shows that in the laboratory there is little difference in any of the nature of the fatty acid used. Sampling shows taste differences, and observations show minor viscosity and colour differences.

**Claims**

1. A composition comprising:
lecithin;
an edible oil;
a fatty acid,
the ratio of fatty acid to lecithin in the composition being such that the composition has the ability to render a hydrophobic surface hydrophilic.

2. A composition as claimed in Claim 1 in which the ratio of free fatty acid to plastic lecithin is within the range 4.5:1 to 11:1.

3. A composition as claimed in Claim 1 or 2 in which the lecithin is derived from soy bean.

4. A composition as claimed in Claim 1 or 2 in which the lecithin is derived from cotton seed, eggs or groundnut.

5. A composition as claimed in any one of Claims 1 to 4 in which the edible oil is a fish or a vegetable oil.

6. A composition as claimed in any one of Claims 1 to 4 in which the edible oil is codliver oil.

7. A composition as claimed in any one of Claims 1 to 6 in which the fatty acid is myristic acid.

8. A composition as claimed in any one of Claims 1 to 6 in which the fatty acid is lauric, palmitic or stearic acid.

9. A composition as claimed in any one of Claims 1 to 8 including a polyhydric alcohol.

10. A composition as claimed in Claim 9 in which the polyhydric alcohol is glycerol or propylene glycol.

11. A composition as claimed in any preceding claim including a surfactant.

12. A composition as claimed in Claim 11 in which the surfactant is a non-ionic surfactant.

13. A composition as claimed in Claim 11 in which the surfactant is an ionic surfactant.

14. A composition as claimed in any preceding claim further including a soap, a bacteriostat and/or an anaesthetic.

15. A composition as claimed in any preceding claim further including water.